# EUROPÄISCHE PATENTSCHRIFT

(11) **EP 1 625 909 B1**
(45) Veröffentlichungstag und Bekanntmachung des Hinweises auf die Patenterteilung: **27.04.2016**
(21) Anmeldenummer: 05023573.8
(22) Anmeldetag: 27.05.1998
(51) Int. Cl.: B23D 61/12, B28D 1/12

(54) **Schneidwerkzeug**
Cutting tool
Outil de coupe

(30) Priorität: 28.05.1997 CH 125497; 21.08.1997 CH 195297; 29.10.1997 CH 250297
(43) Veröffentlichungstag der Anmeldung: 15.02.2006
(62) Teilanmeldung aus: 98810492.3
(73) Patentinhaber: maRoc GmbH, 8500 Frauenfeld (CH); C. & E. Fein GmbH, 73529 Schwäbisch-Gmünd-Bargau (DE)
(72) Erfinder: Steiger, Marco, 8524 Uesslingen (CH); Suter, David, 8488 Turbenthal (DE)
(74) Vertreter: Witte, Weller & Partner Patentanwälte mbB

(56) Entgegenhaltungen:
- DE-A1- 2 849 760
- GB-A- 2 107 641
- US-A- 3 905 105
- US-A- 4 454 901
- US-A- 5 016 356
- US-A- 5 265 340
- US-A- 5 306 285
- US-A- 5 507 763

## Beschreibung

Die Erfindung betrifft ein Schneidwerkzeug gemäß dem Oberbegriff des Anspruchs 1.

Ein derartiges Schneidwerkzeug ist aus der US-A-5 507 763 bekannt.

Bei dem bekannten Schneidwerkzeug handelt es sich um ein chirurgisches Sägeblatt, das in Verbindung mit einem oszillierenden Antrieb verwendet werden kann, um beispielsweise Gipsverbände aufzuschneiden. Das Schneidwerkzeug besitzt zur Befestigung an der oszillierenden Abtriebswelle des Oszillationsantriebes einen Schlitz, mit dem es auf die Abtriebswelle aufschiebbar ist. Zur zusätzlichen Befestigung dient eine Reihe von kreisförmigen Öffnungen, die um den Schlitz herum angeordnet sind und mit Hilfe derer durch entsprechende Vorsprünge an der Werkzeugabtriebswelle eine formschlüssige Verbindung herstellbar ist.

Ein weiteres Schneidwerkzeug ähnlicher Art ist aus der US-A-4 513 742 bekannt.

Auch dieses Schneidwerkzeug ist zur Befestigung an einem Oszillationsantrieb ausgebildet und ist mittels eines Schlitzes daran befestigbar.

Aus der DE-A-2 849 760 ist ferner ein Schneidwerkzeug gemäß dem Oberbegriff von Anspruch 1 bekannt.

Aus der US 5,306,285 A und aus der GB 2 107 641 A sind weitere Schneidwerkzeuge in Form von Sägeblättern bekannt, die für oszillierende chirurgische Sägen verwendet werden. Hierbei liegen die Spitzen der Zähne entweder auf einer Kreislinie oder auf einer Geraden.

Vor diesem Hintergrund liegt der Erfindung die Aufgabe zugrunde, ein verbessertes Schneidwerkzeug zum Schneiden oder Sägen anzugeben, das von einer oszillierend angetriebenen Werkzeugabtriebswelle einer Werkzeugmaschine antreibbar ist und mit dem ein möglichst gutes Schneidergebnis erzielbar ist.

Diese Aufgabe wird durch ein Schneidwerkzeug gemäß Anspruch 1 gelöst.

Da erfindungsgemäß die Sägezähne aus zwei oder mehr in einem Winkel zueinander stehenden Geraden liegen, kann eine im Wesentlichen rechtwinklig zum Einstich verlaufende Grundfläche, bzw. eine parallel zur Oberfläche am Stichloch vorgetriebene Schnittfläche erzeugt werden.

Gemäß einer weiteren Ausführung der Erfindung sind im Schneidwerkzeug ein Längsschnitt und/oder seitliche Rücksprünge als Kanäle zur Spanabfuhr ausgebildet. Dies verhindert das Verklemmen des Scheidwerkzeugs zwischen den vom Schneidwerkzeug erzeugten Schnittflächen.

Der Werkzeugvorschub benötigt eine geringe Vorschubkraft, da sich das Werkzeug selbsttätig in das Werkstück einarbeiten kann.

Schnitte lassen sich beispielsweise fluchtend zu einer bestehenden Fläche in eine dazu winklig stehende Fläche erzeugen.

Anhand eines illustrierten Ausführungsbeispieles wird die Erfindung näher erläutert. Es zeigen
- Figur 1: eine Seitenansicht einer Werkzeugmaschine (mit Schneidwerkzeug) mit oszillierender Abtriebswelle,
- Figur 2: einen Grundriss der Werkzeugmaschine in Figur 1,
- Figur 3: eine Aufsicht auf das Schneidwerkzeug und
- Figur 4: einen Längsschnitt längs Linie IV-IV in Figur 3,
- Figur 5: eine Aufsicht auf eine weitere Ausführung eines Schneidwerkzeugs,
- Figur 6: eine Aufsicht auf eine weitere Ausführung eines Schneidwerkzeugs,
- Figur 7: ein weiteres Schneidwerkzeug mit zwei geneigt in einem stumpfen Winkel liegenden Sägezahnreihen.

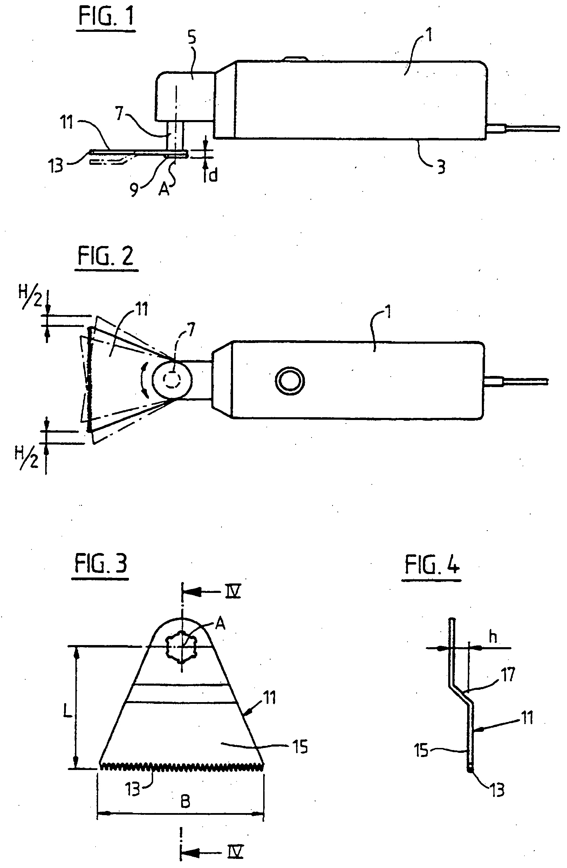

Die in Figur 1 dargestellte motorisch angetriebene und von Hand führbare Werkzeugmaschine 1 weist einen Griffteil 3 mit dem Antriebsmotor und einen Winkelgetriebeteil 5 mit einem darin angeordneten, nicht sichtbaren Winkelgetriebe auf, welches eine nach außen geführte Abtriebswelle 7 trägt, an deren Ende ein Befestigungsmittel 9, z.B. eine Schraube, aufgesetzt ist, mit welcher ein Werkzeug 11 drehfest mit der Abtriebswelle 7 verbindbar ist. Der Antrieb kann elektrisch oder pneumatisch erfolgen. Die Abtriebswelle 7 vollführt eine oszillierende Drehbewegung um die Achse A in einem Bereich von ca. 2 Winkelgraden und mit einer vorzugsweise stufenlos verstellbaren Frequenz von 0 bis zu 20.000 und mehr Hüben pro Minute.

Das Schneidwerkzeug 11 umfasst eine Schneide 13, welche mit einer Zahnung, vorzugsweise mit Kreuzschliff oder geschränktem Schliff, bestückt ist. Das Schneidwerkzeug 11 umfasst im Ausführungsbeispiel eine im Wesentlichen trapezförmige Platte 15, an deren Basis die Schneide 13 ausgebildet ist. Vorzugsweise weist die Platte 15 eine Stufe 17 auf, deren Höhe h mindestens der Dicke d des Befestigungsmittels 9 entspricht. Die Länge L des Schneidwerkzeugs, gemessen vom Drehpunkt der Achse A bis zur Schneide 13 kann je nach Einsatzzweck variieren. Zum Erzeugen tiefer Schnitte muss selbstverständlich eine größere Länge L vorgesehen werden, als für das Anbringen von beispielsweise nicht sehr tiefen Nuten. Eine größere Länge L bewirkt allerdings bei gleichbleibendem Drehwinkel der Werkzeugmaschine 1 einen größeren Hub H/2. Eine optimale Länge L beträgt etwa 80 mm. Die Breite B der Schneide beträgt im Beispiel ca. 60 mm. Sie kann selbstverständlich für die Erzeugung sehr kleiner Einstiche wesentlich kleiner sein und hängt zudem von der Länge L ab.

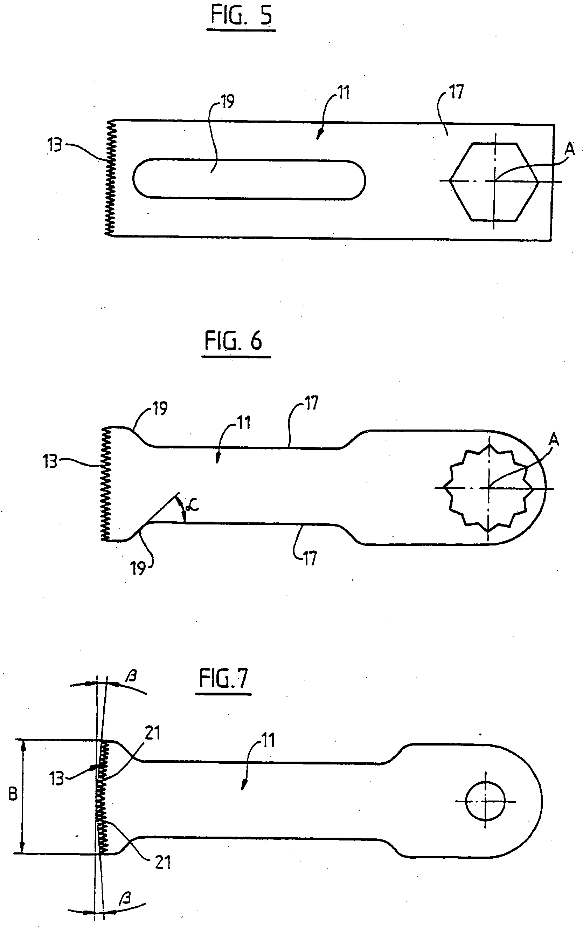

In der Ausgestaltung des nicht erfindungsgemäßen Schneidwerkzeugs 11 nach Figur 5 weist dieses parallel verlaufende Seitenkanten 17 auf. Zur Abfuhr der Späne ist im Zentrum des Schneidwerkzeugs ein Längsschlitz 19 ausgebildet.

In der Ausgestaltung des Schneidwerkzeugs 11 gemäß Figur 6 verlaufen die Seitenkanten 17 ebenfalls parallel, jedoch liegen sie in geringerem Abstand zueinander. Die Verbindung von der Schneide 13 zu den zurückgesetzten Seitenkanten 17 erfolgt durch Verbindungen 19, die in einem Winkel α zu den Seitenkanten 17 verlaufen. Durch die Verschmälerung des Schneidwerkzeugs 11 können die Späne in den verjüngten Bereichen austreten und abgeführt werden.

In einer Ausgestaltung der Erfindung gemäss Figur 7 liegen die Zahnspitzen der Zähne auf der Schneide 13 nicht auf einer Geraden, sondern die Schneide 13 ist in zwei winklig zueinander verlaufende Abschnitte 21 aufgeteilt. Der Winkel zwischen den wiederum auf Geraden liegenden Zahnspitzen in den Abschnitten 21 ist stumpf. Die Winkel β der beiden Abschnitte 21 zu einer wie in den Figuren 2 bis 6 beschriebenen Ausführung betragen beispielsweise 1,5° bis 2°. Bei kurzen Schneidwerkzeugen 11 liegt der Winkel β vorzugsweise über zwei Winkelgraden; bei kürzeren Klingen unter zwei Winkelgraden. Bei einer Klingenlänge von 100 mm, d.h. einem Abstand der Schneide 13 vom Drehpunkt A von 100 mm verwendet man vorzugsweise einen Winkel von 4,6°. Je nach der gewählten Breite B der Schneide 13 kann diese in mehr als zwei Abschnitte 21, die jeweils in einem Winkel β zueinander verlaufen, aufgeteilt sein.

Die Ausbildung der Schneide 13 hängt, wie bereits oben erwähnt, von der Art des Werkstoffes, der zu schneiden ist, ab. Als sehr vorteilhaft hat sich eine Schneide 13 mit einem Kreuzschliff oder geschränktem Schliff erwiesen für Arbeiten in Holz, Gips, z.B. beim Erzeugen von rechteckigen Einstichen am Bau (in Balken), z.B. Zapfenlöcher, wie sie beim Zusammenfügen von Gebälk benutzt werden, zum Erzeugen von Schattenfugen, zum Heraustrennen von auszutauschenden Fenstern, zum Erzeugen von Schlitzen zum Einschieben einer Platte etc. Nicht erfindungsgemäß können bei Arbeiten mit Verbundstoffen aus Kunststoff oder Arbeiten in Stein Industriediamanten in herkömmlicher Weise auf die Schneide 13 aufgebracht werden und kann bei der Bearbeitung von Metall Korund als Schneidmaterial eingesetzt werden. Die Verbindung des Schneidmaterials (Korund, Diamanten etc.) mit der Platte 15 erfolgt in bekannter Weise wie bei der Bestückung von anderen Werkzeugen mit den entsprechenden Schneidmaterialien.

Die Handhabung des Schneidwerkzeugs ist sehr einfach. Muss beispielsweise in einem Raum auf einem bestehenden Boden ein Parkettbelag oder ein anderer Belag verlegt werden und soll dieser unter die Türzargen, die bis zum bestehenden Boden reichen, geführt werden, so lässt sich in einfacher Weise ein nur wenige Millimeter über dem bestehenden Boden liegender Einschnitt und, falls die Türzarge in den bestehenden Boden hineinragt, auch bündig zum Boden ein Einschnitt erzeugen, so dass ein sauberer Schlitz zum Einfügen des neuen Bodens entsteht. Der Einstich, der sich mit dem erfindungsgemäßen Schneidwerkzeug ausführen lässt, kann nicht nur bündig zu einer Fläche, die parallel zur Schneide liegt, erfolgen, sondern der Einstich kann auch in Ecken von Möbeln oder Räumen parallel zur seitlich verlaufenden Wand erfolgen. Es sind folglich keine Nacharbeiten mit Stechbeitel oder dergleichen notwendig. Das Schneidwerkzeug, insbesondere wenn es sich um ein solches mit Kreuzschliff oder geschränktem Schliff handelt, arbeitet sich im Wesentlichen selbstständig, d.h. mit geringstem Druck, in das zu schneidende Material hinein. Die geradlinig verlaufende Schneide bewirkt denn auch, dass der Grund des erzeugten Schlitzes oder Loches bis in die Ecken gleich tief verläuft. Dies im Gegensatz zu bogenförmigen Werkzeugen, mit denen die seitlichen Bereiche immer weniger tief sind und zudem eine nicht entsprechend gleich saubere Schnittkante aufweisen.

## Patentansprüche

1. Schneidwerkzeug mit einer Schneide zum Eingriff mit einem Werkstück und zur Befestigung an einer motorisch angetriebenen, handgeführten Werkzeugmaschine (1), die eine Werkzeugabtriebswelle (7) aufweist, die mit einer oszillierenden Drehbewegung um eine Achse (A) antreibbar ist, wobei das Schneidwerkzeug (11) zur Befestigung auf der Werkzeugabtriebswelle (7) ausgebildet ist, und die Schneide (13) des Schneidwerkzeugs (11) mit einer Zahnung bestückt ist und, wenn das Schneidwerkzeug (11) auf der Werkzeugabtriebswelle (7) befestigt ist, in einem Abstand (L) zur Achse (A) angeordnet ist, wobei die Spitzen der Zähne an der Schneide (13) in mindestens einem Abschnitt auf einer Geraden liegen, **dadurch gekennzeichnet, dass** die Spitzen der Sägezähne der Schneide (13) auf zwei oder mehr in stumpfem Winkel liegenden Abschnitten (21) angeordnet sind, und dass die auf einer Geraden liegenden Spitzen der Sägezähne eines Abschnitts (21) in einem Winkel (β) von 1,5° bis 4,6°, vorzugsweise 1,5° bis 2°, zu den auf einer Geraden liegenden Spitzen der Sägezähne eines benachbarten Abschnitts (21) liegen.

2. Schneidwerkzeug nach Anspruch 1, **dadurch gekennzeichnet, dass** das Schneidwerkzeug (11) eine allseits umschlossene Öffnung zur Befestigung an der Werkzeugabtriebswelle (7) aufweist, durch die ein Befestigungsmittel (9) mit der Werkzeugsantriebswelle (7) verbindbar ist, wobei die Öffnung kreisförmig oder sechskantförmig ausgebildet ist, die im Falle einer sechskantförmigen Öffnung so orientiert ist, dass zwei einander gegenüberliegende Spitzen mit einer Längsachse des Schneidwerkzeuges ausgerichtet sind.

3. Schneidwerkzeug nach einem der vorhergehenden Ansprüche, **dadurch gekennzeichnet, dass** die Schneide (13) an einer Platte (15) ausgebildet oder an dieser befestigt ist.

4. Schneidwerkzeug nach einem der vorhergehenden Ansprüche, **dadurch gekennzeichnet, dass** im Schneidwerkzeug (11) ein Längsschlitz (19) und/oder seitliche Rücksprünge als Kanäle zur Spanabfuhr ausgebildet sind.

## Claims

1. A cutting tool comprising a cutting edge for engaging a workpiece and for securing on a motor-driven hand-held tool machine (1), comprising a tool output shaft (7) which can be driven with an oscillating rotary motion about an axis (A), wherein the cutting tool (11) is configured for securing on the tool output shaft (7), and wherein the cutting edge (13) of the cutting tool (11) is equipped with a toothing, and, when the cutting tool (11) is fixed to the tool output shaft (7), is arranged at a distance (L) to the axis (A), wherein the tips of the teeth are arranged at the cutting edge (13) along a straight line at least in one section, **characterized in that** the tips of the teeth of the cutting edge (13) are arranged on two or more sections (21) arranged at a blunt angle, and **in that** the tips of the sawing teeth of one section (21) lying on a straight line are arranged at an angle (β) of 1.5° to 4.6°, preferably 1.5° to 2°, to the tips of the sawing teeth of the adjacent section (21) lying on a straight line.

2. The cutting tool of claim 1, **characterized in that** the cutting tool (11) comprises an opening enclosed on all sides for securing on the tool output shaft (7), through which a securing means (9) is connectable with the tool output shaft (7), wherein the opening is configured circularly or hexagonally being oriented in the case of a hexagonal shape such that two tips opposing each other are aligned with a longitudinal axis of the cutting tool.

3. The cutting tool of any of the preceding claims, **characterized in that** the cutting edge (13) is arranged at a plate (15) or is attached thereto.

4. The cutting tool of any of the preceding claims, **characterized in that** within the cutting tool (11) an elongated slot (19) and/or lateral offsets are configured as channels for chip removal.

## Revendications

1. Outil de coupe comprenant une lame destinée à venir en prise avec une pièce et à être fixée à une machine-outil (1) manuelle à entraînement motorisé, laquelle possède un arbre d'entraînement d'outil (7) qui peut être entraîné avec un mouvement rotatif oscillant autour d'un axe (A), l'outil de coupe (11) étant configuré pour être fixé sur un arbre d'entraînement d'outil (7) et la lame (13) de l'outil de coupe (11) étant pourvue d'une denture et, lorsque l'outil de coupe (11) est fixé sur l'arbre d'entraînement d'outil (7), disposée à une distance (L) de l'axe (A), les pointes des dents sur la lame (13) se trouvant sur une droite au moins dans une portion, **caractérisé en ce que** les pointes des dents de scie de la lame (13) sont disposées sur deux portions (21) ou plus orientées selon un angle obtus, et **en ce que** les pointes disposées sur une droite des dents de scie d'une portion (21) sont orientées selon un angle (ß) de 1,5° à 4,6°, de préférence de 1,5° à 2°, par rapport aux pointes disposées sur une droite des dents de scie d'une portion (21) voisine.

2. Outil de coupe selon la revendication 1, **caractérisé en ce que** l'outil de coupe (11) possède une ouverture fermée de tous les côtés destinée à la fixation à l'arbre d'entraînement d'outil (7), à travers laquelle un moyen de fixation (9) peut être relié à l'arbre d'entraînement d'outil (7), l'ouverture étant de configuration circulaire ou hexagonale et, dans le cas d'une ouverture hexagonale, est orientée de telle sorte que deux pointes mutuellement opposées sont alignées avec un axe longitudinal de l'outil de coupe.

3. Outil de coupe selon l'une des revendications précédentes, **caractérisé en ce que** la lame (13) est configurée sur une plaque (15) ou fixée à celle-ci.

4. Outil de coupe selon l'une des revendications précédentes, **caractérisé en ce qu'**une fente longitudinale (19) et/ou des retraits latéraux sont formés dans l'outil de coupe (11) en tant que canaux pour l'évacuation des copeaux.
